Europäisches Patentamt

European Patent Office

Office européen des brevets

(19)

(11) **EP 1 369 097 A1**

(12) # EUROPEAN PATENT APPLICATION

(43) Date of publication:
**10.12.2003 Bulletin 2003/50**

(51) Int Cl.⁷: **A61F 2/06**, A61M 25/10

(21) Application number: **02253985.2**

(22) Date of filing: **07.06.2002**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI**<br><br>(71) Applicant: **CathNet-Science Holding A/S**<br>**4000 Roskilde (DK)** | (72) Inventor: **Van der Leest, Machiel**<br>**75014 Paris (FR)**<br><br>(74) Representative: **Boydell, John Christopher**<br>**Stevens, Hewlett & Perkins**<br>**Halton House**<br>**20/23 Holborn**<br>**London EC1N 2JD (GB)** |

(54) **Balloon catheter assembly**

(57)     A balloon catheter assembly comprising twin balloons (6,7) for the treatment of bifurcated vessels. One or both balloons (6,7) are stepped to provide a proximal section (33), a distal section (34) of larger diameter, and a step (35) between the two. In use, the twin balloons are positioned across the bifurcation (36) so that the distal sections (34) of the two balloons enter respectively the main and side branches (38,39) distally of the bifurcation, and the smaller diameter proximal sections (33) are positioned side by side in the common area (40) in the vessel (37) proximally of the bifurcation. This design can avoid over-inflation in the common area (40), due to the presence of two balloons within the lumen of the vessel (37).

Figure 3

EP 1 369 097 A1

**Description**

This invention relates to a balloon catheter assembly.

Balloon catheters are used in the treatment of various anatomical ducts in the body, such as blood vessels, the urinary ducts, or digestive ducts. A particular application is in the treatment of blood vessels exhibiting stenosis.

The conventional balloon catheter comprises a catheter delivery tube on the distal end of which is mounted a balloon of flexible material. During a balloon angioplasty procedure, the balloon is moved along blood vessels within the body to the site of the lesion and, when correctly positioned, is inflated, usually by means of fluid pressure supplied along the hollow interior of the catheter delivery tube. As the balloon inflates it expands against the vessel wall and thus applies outwards pressure to the wall tending to enlarge the vessel's lumen to remove or reduce the effect of a partly blocked vessel. Once the balloon has been inflated to the desired extent, the pressure is released, and the balloon deflates and is removed, leaving the vessel in its enlarged state. In order to avoid collapse of the vessel after treatment, a stent may optionally be carried by the balloon and left in place when the balloon is removed in order to act as a reinforcement for the vessel wall, thus maintaining the enlarged state of the lumen. There are many designs of stent available and their construction and deployment are both well known.

The present invention is directed particularly at the treatment of bifurcated vessels, these usually comprising a main branch and a side branch, and wherein treatment is necessary in both branches, close to the bifurcation, and/or at the bifurcation itself. Currently treatment of such vessels would usually be carried out with two separate balloon catheters using a technique known as the kissing balloon technique and described, for example, in US Patent No. 4,896,670 and in WO 00/27307. Other techniques, for example using a stent to initially dilate the main branch of the vessel, may also be used.

The kissing balloon technique utilises paired balloons which are passed together to the treatment site. One of the balloons is intended to enter the side branch, whilst the other will continue along the main branch. If the treatment site is at or near the bifurcation then the paired balloons will lie across the bifurcation and thus, on one side of the bifurcation, there is a common part in which both balloons will be present within the vessel. Generally, the side branch will have a smaller diameter than the main branch and the main branch will have more or less the same diameter on both sides of the bifurcation (in practice the diameter on the distal side of the bifurcation may be slightly smaller than that on the proximal side). The result of this is that the paired balloons in the common part are liable to overstretch the vessel in the common part when the balloons are inflated during the surgical procedure.

The present invention seeks to overcome this problem by providing that at least one of the two balloons is of stepped construction.

In accordance with the invention there is provided a balloon catheter assembly comprising a catheter delivery system at the distal end of which is mounted at least two expandable balloons, said balloons being of a generally cylindrical construction having a proximal section and a distal section and wherein, in the case of at least one of said balloons, the diameter of the proximal section is less than that of the distal section.

This stepped construction of balloon enables those parts of the balloons which pass into the main and side branches beyond the bifurcation to be tailored for the particular circumstances in the branches, whilst enabling those parts of the balloons which lie in the common part, where the two balloons are side by side, to be likewise tailored to reduce the risk of over-stretching the vessel in this area.

To achieve this, the total circumference of the two balloons in the common part must be reduced over what it would have been had both balloons been of a constant diameter along their length. Making one or both balloons of smaller diameter in the common part enables this to be realised. Since, as already mentioned, it is often the case that the diameter of the main branch does not significantly change as between one side of the bifurcation and the other, then the total circumference of the proximal sections of both balloons in the common part will be made approximately equal to the circumference of the distal section of that balloon which has the larger distal section, this latter being the balloon which enters the main branch beyond the bifurcation. The word "approximately" is used here because, in practice, a correction factor has to be applied to the calculation to take into account the fact that, in the common part, expansion of the two side-by-side balloons might result in a flattened section between them where they are in contact. This means that, in practice, the total circumferential length of the two proximal sections lying in the common part should be slightly greater than the diameter of the large distal section.

The relative length of the distal section and proximal section of the stepped balloons may be adjusted according to the particular requirements of use. In practice, the step between the smaller diameter proximal section and the larger diameter distal section may be positioned in the approximate centre of the longitudinal length of the balloon, but this is not necessarily the case. If both balloons are stepped, then the longitudinal position of the step will normally be approximately the same as between the two balloons, but this is not necessarily the case.

In the embodiment of the invention to be described below, the two balloons are substantially identical, both being stepped, with equal diameters in their respective proximal and distal sections. However, as already mentioned, the relative diameters can be tailored according to the circumstances of use: for example the balloon intended to enter the side branch, even if it is of stepped construction, may have distal section of smaller diameter than the distal section of the other balloon. The corresponding proximal sections may be of identical diameters, or they may be different.

It will be understood that, for the balloon catheter assembly of the present invention to operate satisfactorily, it must be reasonably accurately positioned at the treatment site. In particular the proximal sections of the two balloons should lie in the common part, immediately proximal to the bifurcation, whilst the distal sections of the two balloons should enter the side and main branches respectively. Thus the step between the two sections should be positioned approximately at the bifurcation itself. In order to assist the surgeon in positioning the balloons, ratio-opaque marker rings may be used. These are well known in the art and will not be described in detail. The rings are normally fitted close to the proximal and/or distal ends of the balloons, but a further ring, corresponding in position to the step, may be fitted in addition to or instead of the existing rings to allow for precise positioning of the proximal and distal sections of the or each balloon relative to the bifurcation, as explained above.

The step between the smaller proximal section and the larger distal section of the balloon, may be formed as a sharp step, but is preferably formed as a gradual transition from the smaller diameter to the larger diameter. Such transition may be of straight sided cone shape, or may be convex or concave. Operationally, there may be advantage in making the step of one balloon of a concave profile, and that of the other of a corresponding convex profile so that they locate together.

It is well known that the balloons are inflated using fluid pressure applied along a catheter delivery system of some type. This may comprise a catheter delivery tube for each balloon, or a single catheter delivery tube which is branched at its distal end into two branch tubes, each of which carries a respective balloon. Generally speaking each balloon is mounted at the distal end of the respective branch delivery tube.

During a surgical procedure, the two balloons are moved simultaneously to the area to be treated. For this purpose, one or more guide wires may be used, as will be explained in more detail below, and the balloons themselves are generally brought together so as to present the minimum cross sectional area for the journey to the treatment site.

The balloons may each be positioned at the same distance from the branch along their respective branch delivery tubes so that the balloons are essentially in parallel with one another. Typically the distance between the branch and the proximal ends of the balloons will be between 5 mm and 50 mm. Alternatively the balloons may be positioned such that they are positionally stepped in relation to one another.

It has already been mentioned that the different dimensions of the two balloons may be adjusted according to the anticipated circumstances of use. In addition to this, the two balloons may have a different compliance so as to give them different inflation characteristics; this might be desirable where, for example, a layer of plaque on one of the branches of the vessel to be treated has to be broken, this requiring an excess fluid pressure to be applied to the balloon in the branch concerned. If such an increased pressure were also to be applied to the other balloon, then that balloon might become over-inflated and cause damage to the vessel wall. One way of overcoming this problem is to use independent fluid pressure supplies to the two balloons (see below); however, if the two balloons are supplied from a common supply, an alternative way of dealing with the problem is to make the other balloon of a construction which is less compliant so that, for a given fluid pressure, it inflates to a lesser extent. So-called "non-compliant" balloons are also available which expand, under pressure, up to a given diameter but will not expand significantly further even if the pressure is increased further (subject, of course, to extreme excess pressures being employed).

Variation of compliance can be achieved either by suitable selection of the material of the balloon, or by changing the thickness and other physical characteristics of the balloon, or a combination of both.

For operational reasons it is preferred that the distal end of one of the balloons extends beyond that of the other so that, as the catheter assembly is moved towards the treatment site, the distal end of one of the balloons extends beyond that of the other. This can be achieved by appropriate positioning of the balloons on their respective branch delivery tubes, or by appropriate sizing of the balloons, or both. In an embodiment, each of the balloons is formed with a narrow distal end beyond the distal section, one of which ends is longer than the other or others so that the distal tip of the corresponding balloon extends beyond the other or others. This elongated distal end may be rounded at the tip to smooth the passage of the catheter assembly to the treatment site, and to allow positioning of the balloon once there.

The or each stent delivery tube is hollow to provide for inflation of the balloons from an appropriate fitting at its proximal end. In the construction in which a common catheter delivery tube branches, the hollow interior of the tube may simply divide into the branch delivery tubes so that the respective balloons may be simultaneously expanded by fluid pressure applied along the catheter delivery tube in the known manner and into the branch delivery tubes. However, it may be necessary to provide for independent inflation of the balloons and, to this end, the hollow interior of the common catheter delivery tube may be divided into two or more independent lumens each of which, at the branch, communicates with a respective one of the branch delivery tubes. In this arrangement each balloon may be inflated independently of the other, and not necessarily at the same time.

It is well known that a guide wire is generally used to guide a balloon catheter to the treatment site. The balloon catheter assembly of the present invention can also use one or more guide wires for this purpose. Various configurations can be used, for example both balloons over-the-wire or one balloon over-the-wire, the other monorail. The guide wire may extend from a fitting at the proximal end of the catheter delivery tube, or may be introduced at some intermediate position, as is well known. In the case of a common catheter delivery tube, the guide wire may be introduced into the catheter delivery tube at an intermediate position proximal of the branch, or may be introduced into one or both of the branch delivery tubes at some position between the branch and the respective balloon. It is also possible to pre-mount a guide wire within one of the balloons; preferably such a pre-mounted guide wire has a tip which is larger than the guide wire lumen in order to prevent the guide wire from retracting into the lumen. Preferably this tip is rounded or has a higher distal profile and, if fitted on a balloon which extends beyond the other or others, can act as a rounded balloon tip to assist movement of the catheter assembly to the treatment site, as mentioned above.

The pre-mounted guide wire is preferably fixed on the exterior of the catheter delivery tube for packing and transportation purposes, for example by means of silicone O-rings.

Once it enters the catheter, whether at the proximal end or at an intermediate position, the guide wire is located in a lumen within the catheter. This lumen may be formed by the passageway through which pressurised fluid is passed to inflate the balloons, or may be a dedicated lumen.

It will thus be seen that there are various different ways in which the catheter assembly of the invention could use guide wires, and some of these will be described, by way of example, below.

One or both of the balloons may be fitted with a stent which is preferably pre-mounted. Alternatively, a single bifurcated stent may be used, one arm being fitted over each of the balloons. Such bifurcated stents are known in the art.

In order that the invention may be better understood, several embodiments thereof will now be described by way of example only and with reference to the accompanying drawings in which:-

Figures 1, 2, 4 and 5 each illustrate diagrammatically an embodiment of the invention;
Figure 3 is a view of the arrangement of Figure 2 fitted within a bifurcated vessel to be treated; and
Figure 6 is a view of the arrangement of Figure 2 fitted with a stent.

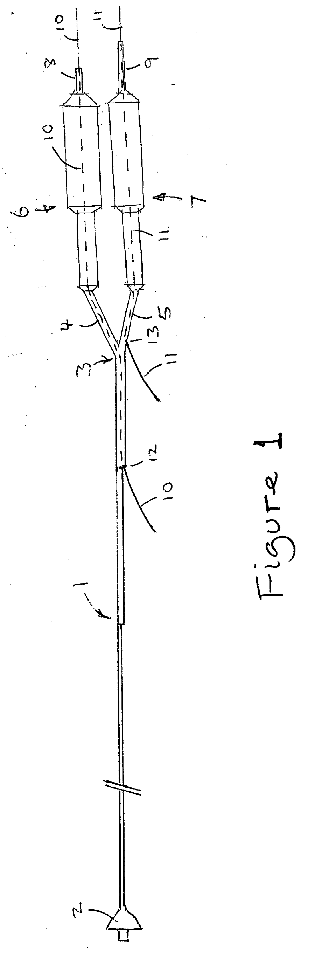

Referring firstly to Figure 1 there is shown a balloon catheter assembly constructed in accordance with the invention. The assembly comprises a catheter delivery tube 1 having, at its proximal end, a conventional fitting, represented diagrammatically under reference 2. At the distal end 3 of the delivery tube 1, the tube branches to form two branch delivery tubes 4,5 which may or may not be the same cross sectional shape as the main delivery tube 1. Mounted on each of the branch delivery tubes 4,5 is a respective inflatable balloon 6,7. For the purpose of illustration, the balloons are shown in a part-inflated state. The construction of the balloons, and the manner of their mounting on the branch delivery tubes is not described in detail since it is well known; each of the balloons is capable of being inflated by means of fluid pressure supplied via lumens in the main delivery tube and the respective branch delivery tube using techniques which are well known. The exact arrangement of these lumens will depend upon the balloon inflation requirements. If the balloons are to be inflated simultaneously, then fluid pressure for this purpose can be supplied by a single lumen formed in the main delivery tube 1, which branches into respective lumens formed in the branch delivery tubes 4,5. If independent balloon inflation is required, separate lumens will have to be provided in the main delivery tube 1, which separate lumens communicate with the individual lumens formed in the branch delivery tubes 4,5.

The main body of each of the balloons, namely the proximal and distal tapered portions and the stepped cylindrical central portion, are substantially identical, although this need not necessarily be the case. However, the narrowed distal end sections 8,9 of the balloons are of different length, that for balloon 7 being longer. The reason for this will be explained below.

Two guide wires 10,11 are used to guide the catheter assembly to the treatment site. The technique is well known and will not be described in detail. During the procedure, each of the guide wires is passed up a respective branch of the vessel to be treated. The guide wires pass through their respective balloons and into a lumen in the respective branch delivery tubes 4,5. The guide wire 10 then passes into a lumen in the main delivery tube 1 and emerges at point 12. The guide wire 11 does not pass beyond the branch, and emerges instead from the branch delivery tube 5 at point 13. Other arrangements are possible: for example both guide wires 10 and 11 could extend into the main delivery tube 1 and emerge at the same or different points.

The stepped construction of each of the balloons 6,7 will now be described with particular reference to Figure 2, which shows the distal part of the catheter assembly of Figure 1 in enlarged detail.

Each balloon comprises a proximal tapered section 30 extending from the distal end of the respective branch delivery tube 4,5 and a distal tapered section 31 extending to a respective distal end section 8,9. The profile of the tapered sections 30,31 may be conical, as shown, or may be concave or convex.

Extending between the tapered sections 30,31 is a central cylindrical section 32 which is divided longitudinally into a proximal section 33 having a diameter Ø1 or Ø2 and a distal section 34 having a larger diameter Ø3 or Ø4. In the embodiment illustrated, the diameter Ø1 of the proximal section 33 of balloon 6 is approximately equal to the diameter Ø2 of the proximal section 33 of balloon 7. Likewise the two diameters Ø3 and Ø4 are approximately equal. However, it is not necessarily the case that the two balloons have equal diameters, and other combinations are possible according to the circumstances.

At the interface between the proximal section 33 and the larger diameter distal section 34 is a step 35 which is preferably a gradual transition, such as shown, between the two diameters. The profile of the step 35 may be conical, as shown, or my be concave or convex.

The dotted line 36 marks the common position of the interface as between the two diameters of the central section of each balloon. During the surgical procedure, the surgeon will seek to position this common interface 36 at the bifurcation in the vessel to be treated. This is illustrated in Figure 3 which shows the twin balloon arrangement of Figure 2 positioned in a vessel 37 which is bifurcated to form a main branch vessel 38 and a side branch vessel 39. The most common situation is illustrated, namely with the side branch vessel 39 being of smaller diameter than the main branch vessel 38, and with the diameter of the main branch vessel 38 being approximately the same as that of the vessel 37.

It will be seen that the interface line 36 has been positioned approximately at the point of bifurcation and this thus defines two parts: to the left of the line 36 in Figure 2 there is a proximal common part 40 in which both balloons 6 and 7 share the same lumen within vessel 37; and to the right of line 36 in Figure 2 is a distal part 41 in which the individual balloons 6,7 have passed along respective branch vessels 38,39. It will further be seen that the common part 40 is mainly occupied by the smaller diameter proximal sections 33 of the balloons 6,7, whilst the distal part 41 is mainly occupied by the larger diameter distal sections 34 of the balloons 6,7.

In order to avoid overstretching of the vessel 37 in the common part, the total circumference of the two proximal sections 33 should be approximately equal to the diameter of each of the distal sections 34.

Mathematically, this can be expressed as:-

$$\text{TT}/2\,(\text{Ø1} + \text{Ø2}) + C(\text{Ø}_1 + \text{Ø}_2) \approx \text{Tr}\text{Ø}_{3/4}$$

simplifying:

$$(1/2 + C/\text{TT}).\,(\text{Ø}_1 + \text{Ø}_2) \approx \text{Ø}_{3 \text{ or } 4}$$

A correction factor C ($\leq$1) has been applied to the above equation to cater for the fact that, in the common part, the two balloons may flatten where they touch during inflation. The value of C will depend upon the inflation pressure and the compliance of the balloon material.

During inflation, both proximal and distal sections 33,34 of balloons 6,7 expand. As will be clear from Figure 3, the distal sections 37 will expand the wall of their respective branch vessel 38,39. At the same time, the wall of the vessel 37 in the common part 40 will be expanded jointly by the two proximal sections 33. Provided that the total circumference of the proximal sections is in line with the equation given above, the inflation force will be approximately the same in the common part 40 as it is in the distal part 41 of the main branch vessel 38. Thus the danger of over-inflating the vessel 37 in the area of the common part 40 is greatly reduced.

Figure 4 illustrates an alternative embodiment in which the distal tip of the longer narrowed portion 9 of balloon 7 is formed with a rounded ball 14. Other details are the same, except that it will be noted that only a single guide wire, reference 15, is used, this extending through the balloon 6 and a lumen in branch delivery tube 4, to a lumen in main delivery tube 1.

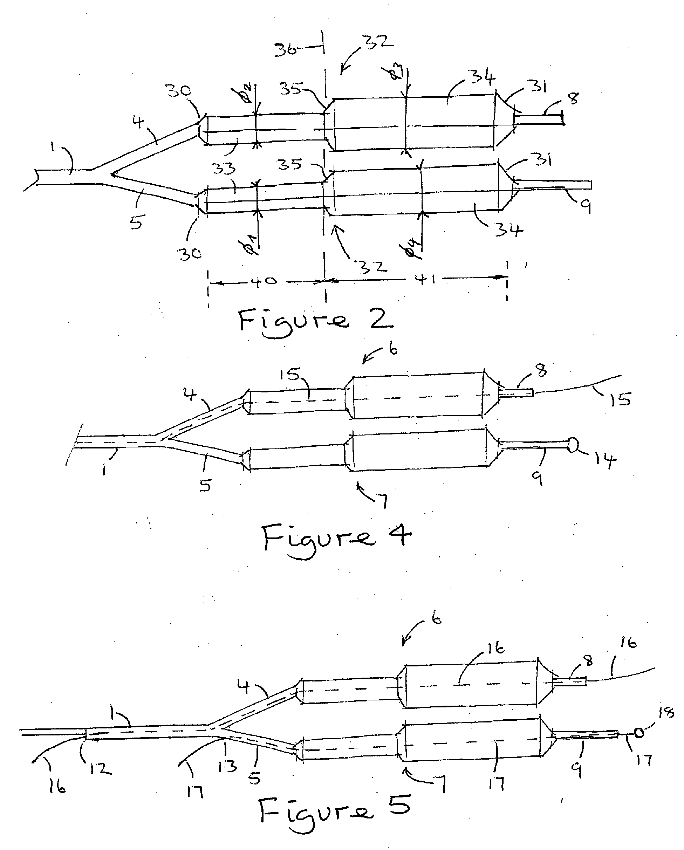

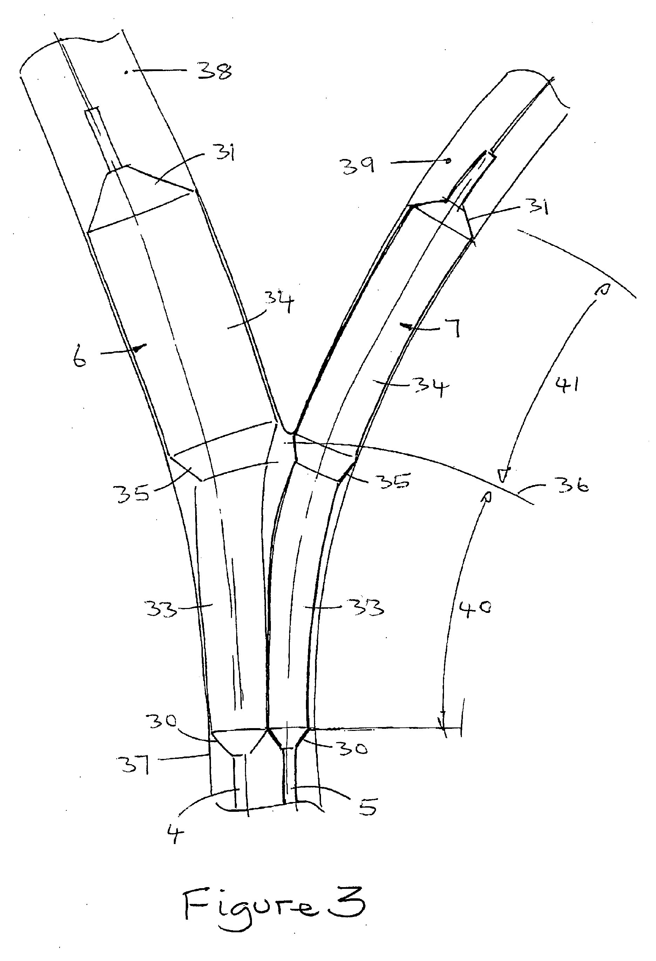

Figure 5 illustrates a similar arrangement in which the balloon 7 is pre-fitted with a guide wire 17 which has an enlarged end 18 to prevent it from retracting back into the lumen. The enlarged end 18 is conveniently round, as shown, so as to act in the same way as the rounded ball 14 in the embodiment of Figure 2. The pre-fitted guide wire emerges from the branch tube 5 at point 13, in a similar manner to the arrangement shown in Figure 1, however the guide wire 17 is different in that it travels with the catheter assembly as it is moved to the treatment site. The guide wire 16 is the same as guide wire 10, and is used in the conventional way.

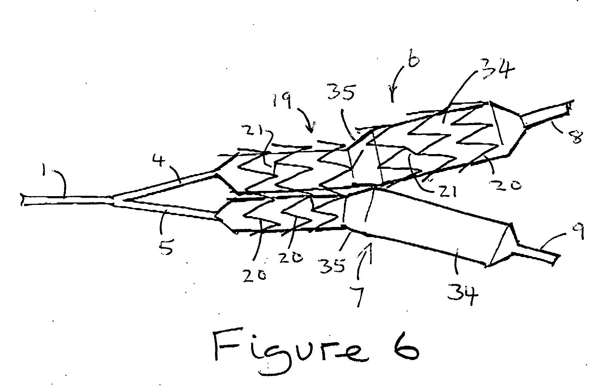

Figure 6 illustrates how a single stent 19 may be fitted to the catheter assembly illustrated in Figures 1 to 3. Any suitable type of stent can be used; that illustrated is a standard stent comprising a series of longitudinally-arranged expandable cylindrical elements 20 which are linked together by linking members 21. Such a stent is described, for example in our copending application No. 0121381.8 and is characterised, amongst other things, by having just a single linking member 21 between each cylindrical element 20. The existence of just a single linking member 21 between elements 20 makes this stent particularly suitable for use with the balloon assembly of the present invention. In Figure 6, the stent is arranged such that the leftmost three cylindrical elements 20 encircle the proximal sections 33 of both balloons 6 and 7, whereas the remaining three cylindrical elements 20 encircle the distal section 34 of balloon 6. It will be understood by those skilled in the art that the stent is crimped or otherwise fitted to the balloons for its journey to the treatment site.

The manner in which the above-described catheter assemblies is used will be apparent to those skilled in the art. In the embodiment of Figure 1 the procedure commences by insertion of the guide wires 10 and 11. The balloon 7, having a longer narrowed distal portion 9, will navigate first into one or the other of the branches of the bifurcated vessel to be treated. The balloon 6 will navigate into the remaining branch. Once in position, the balloons are preferably inflated simultaneously to thereby dilate the two branches at the same time. This will prevent dissection and/or occlusion of one of the branches.

In the embodiment of Figure 4, just a single guide wire 15 is inserted and taken up one of the branches of the bifurcated vessel to be treated. This is then used to guide the catheter assembly to the treatment site with the balloon 6 entering the branch in which the guide wire is situated. The rounded tip 14 enables the balloon 7 to navigate by itself into the other branch.

In the embodiment of Figure 5, the single guide wire 16 is inserted and is then used to guide the catheter assembly to the bifurcation in the vessel being treated. At this point the pre-mounted guide wire 17 can be navigated into one of the branches of the vessel and the catheter advanced to guide the balloon 7 into that branch. Finally, the balloon 6 is navigated into the other branch.

All of the above methods can be used where one or both balloons are pre-fitted with stents. In the case where a bifurcated stent is used, it is possible that one of the "branches" of the stent is shorter than the other. This is not an essential feature of a bifurcated stent but will depend upon conditions at the treatment site. Normally such a stent will be used with the longer branch of the stent in the main branch of the vessel being treated, while the shorter branch of the stent enters a side branch.

**Claims**

1.  A balloon catheter assembly comprising a catheter delivery system at the distal end of which is mounted at least two expandable balloons, said balloons being of a generally cylindrical construction having a proximal section and a distal section and wherein, in the case of at least one of said balloons, the diameter of the proximal section is less than that of the distal section.

2.  A balloon catheter assembly as claimed in claim 1 wherein the total circumference of the proximal sections of the two balloons is approximately equal to the circumference of the distal section of one of the balloons.

3.  A balloon catheter assembly as claimed in claim 2 wherein the total circumference of the proximal sections of the two balloons is approximately equal to the circumference of the distal section of that balloon whose distal section has the largest diameter or, in the case where the distal section diameters are the same, to the circumference of either one of the distal sections.

4.  A balloon catheter assembly as claimed in any one of claims 1, 2 or 3 wherein, in the case of both balloons, the diameter of the proximal section is less than that of the distal section of the same balloon.

5.  A balloon catheter assembly as claimed in any one of the preceding claims wherein the diameter of the distal section of one of the balloons is different to that of the other balloon.

6.  A balloon catheter as claimed in any one of the preceding claims wherein, where the proximal and distal sections are of different diameter, they are separated by a step defining a gradual transition from the smaller diameter of the proximal section to the larger diameter of the distal section.

7.  A balloon catheter as claimed in claim 6 wherein the step has the profile of a cone.

8.  A balloon catheter as claimed in claim 6 wherein the step has a convex profile.

9.  a balloon catheter as claimed in claim 6 wherein the step has a concave profile.

10. A balloon catheter assembly as claimed in any one of the preceding claims wherein each of the balloons have a different compliance so as to give them different inflation characteristics.

11. A balloon catheter assembly as claimed in any one of the preceding claims wherein the distal end of one of the balloons extends beyond that of the other.

12. A balloon catheter assembly as claimed in claim 11 wherein each of the balloons is formed with a narrow distal end section, one of which is longer than the other so that the distal tip of the corresponding balloon extends beyond the other.

13. A balloon catheter as claimed in claim 12 wherein said one of the narrow distal ends is rounded at its distal tip.

14. A balloon catheter assembly as claimed in any one of the preceding claims wherein said catheter delivery system comprises a single catheter delivery tube which is branched at its distal end into at least two branch delivery tubes, each of which carries a respective one of said expandable balloons.

15. A balloon catheter assembly as claimed in claim 14 wherein each balloon is positioned on its respective branch delivery tube the same distance from the branch as the other.

**16.** A balloon catheter as claimed in either one of claims 14 or 15 wherein the stent delivery tube has a hollow interior which divides into the branch delivery tubes so that the respective balloons may be simultaneously expanded by means of fluid pressure applied along the catheter delivery tube.

**17.** A balloon catheter assembly as claimed in either one of claims 14 or 15 wherein the interior of the catheter delivery tube is divided into two independent lumens each of which communicates with a respective one of the branch delivery tubes so that the respective balloons can be expanded independently of one another.

**18.** A balloon catheter assembly as claimed in any one of the preceding claims further comprising at least one guide wire, located within a lumen or lumens within the catheter delivery system, so as to enable the balloons to be guided to a treatment site.

**19.** A balloon catheter assembly as claimed in claim 18 wherein a separate guide wire is used for each balloon.

**20.** A balloon catheter assembly as claimed in either one of claims 18 or 19 wherein one of said guide wires is pre-mounted so as to enable it to be carried to the treatment site with its corresponding balloon.

**21.** A balloon catheter assembly as claimed in claim 20 wherein the distal tip of said pre-mounted guide wire is larger than the corresponding guide wire lumen so as to prevent the guide wire from retracting into the lumen.

**22.** A balloon catheter assembly as claimed in any one of the preceding claims wherein one or both balloons are fitted with a stent.

**23.** A balloon catheter assembly as claimed in claim 22 wherein a single stent is used for both balloons, said stent comprising a series of longitudinally spaced expandable elements, and wherein a predetermined number of the elements at the proximal end of the stent extend over both balloons, and the remaining elements cover just one of the balloons.

**24.** A balloon catheter assembly as claimed in any one of claims 1 to 22 wherein said balloons are fitted with a bifurcated stent having two arms, each positioned over a respective balloon.

Figure 1

Figure 2

Figure 4

Figure 5

Figure 3

Figure 6

European Patent
Office

## EUROPEAN SEARCH REPORT

Application Number

EP 02 25 3985

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99 44539 A (SCIMED LIFE SYSTEMS INC) 10 September 1999 (1999-09-10)<br><br>* page 5, line 20 - page 7, line 6 *<br>* page 25, line 3 - line 18; figures *<br>--- | 1-9, 11-15, 17-22 | A61F2/06<br>A61M25/10 |
| A | WO 00 44307 A (SCIMED LIFE SYSTEMS INC) 3 August 2000 (2000-08-03)<br><br>* page 7, line 19 - page 8, line 30 *<br>* page 12, line 30 - page 16, line 27; figures *<br>--- | 1,5,6, 14-19, 22-24 | |
| A | WO 99 24104 A (AVE CONNAUGHT) 20 May 1999 (1999-05-20)<br><br>* abstract; figures *<br>--- | 1,14-16, 18-20, 22-24 | |
| A | US 6 056 721 A (SHULZE JOHN E) 2 May 2000 (2000-05-02)<br>* column 6, line 47 - line 49 *<br>* column 7, line 44 - line 45; figure 2 *<br>--- | 1,10 | TECHNICAL FIELDS SEARCHED (Int.Cl.7)<br><br>A61F<br>A61M |
| A | US 2002/022874 A1 (WILSON W STAN) 21 February 2002 (2002-02-21)<br><br>* page 3, paragraph 36 - page 4, paragraph 40; figures *<br>----- | 1,11,14, 15, 17-19, 22-24 | |

The present search report has been drawn up for all claims

| Place of search | Date of completion of the search | Examiner |
|---|---|---|
| THE HAGUE | 8 April 2003 | Kousouretas, I |

EPO FORM 1503 03.82 (P04C01)

**EP 1 369 097 A1**

## ANNEX TO THE EUROPEAN SEARCH REPORT
## ON EUROPEAN PATENT APPLICATION NO.

EP 02 25 3985

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2003

| Patent document cited in search report | | Publication date | Patent family member(s) | | Publication date |
|---|---|---|---|---|---|
| WO 9944539 | A | 10-09-1999 | US | 6099497 A | 08-08-2000 |
| | | | CA | 2321040 A1 | 10-09-1999 |
| | | | EP | 1065994 A2 | 10-01-2001 |
| | | | JP | 2002505148 T | 19-02-2002 |
| | | | WO | 9944539 A2 | 10-09-1999 |
| WO 0044307 | A | 03-08-2000 | CA | 2360551 A1 | 03-08-2000 |
| | | | EP | 1152711 A1 | 14-11-2001 |
| | | | WO | 0044307 A1 | 03-08-2000 |
| WO 9924104 | A | 20-05-1999 | AU | 1170199 A | 31-05-1999 |
| | | | EP | 0951310 A1 | 27-10-1999 |
| | | | WO | 9924104 A1 | 20-05-1999 |
| | | | US | 6142973 A | 07-11-2000 |
| US 6056721 | A | 02-05-2000 | AU | 740657 B2 | 08-11-2001 |
| | | | AU | 8698198 A | 01-03-1999 |
| | | | EP | 1005382 A1 | 07-06-2000 |
| | | | JP | 2001513374 T | 04-09-2001 |
| | | | WO | 9907432 A1 | 18-02-1999 |
| US 2002022874 | A1 | 21-02-2002 | US | 6254593 B1 | 03-07-2001 |
| | | | AU | 4712001 A | 25-06-2001 |
| | | | WO | 0143665 A2 | 21-06-2001 |
| | | | US | 2002111675 A1 | 15-08-2002 |